# EUROPÄISCHE PATENTSCHRIFT

(11) **EP 0 731 675 B1**
(45) Veröffentlichungstag und Bekanntmachung des Hinweises auf die Patenterteilung: **29.04.1998**
(21) Anmeldenummer: 95903791.2
(22) Anmeldetag: 02.12.1994
(51) Int. Cl.: A61F 2/30, G05B 19/40, G06T 17/40, G06F 15/00

(54) **VERFAHREN ZUR HERSTELLUNG VON ENDOPROTHESEN**
PROCESS FOR PRODUCING ENDOPROSTHESES
PROCEDE DE FABRICATION D'ENDOPROTHESES

(30) Priorität: 04.12.1993 DE 4341367
(43) Veröffentlichungstag der Anmeldung: 18.09.1996
(73) Patentinhaber: Eufinger, Harald, 44803 Bochum (DE); Heuser, Lothar, 44797 Bochum (DE); Kruse, Dieter, 58455 Witten (DE); Machtens, Egbert, 58300 Wetter (DE); Seifert, Hans, 58509 Lüdenscheid (DE)
(72) Erfinder: Eufinger, Harald, 44803 Bochum (DE); Heuser, Lothar, 44797 Bochum (DE); Kruse, Dieter, 58455 Witten (DE); Machtens, Egbert, 58300 Wetter (DE); Seifert, Hans, 58509 Lüdenscheid (DE)
(74) Vertreter: Behrendt, Arne, Dipl.-Ing.
(86) Internationale Anmeldenummer: EP9404020
(87) Internationale Veröffentlichungsnummer: WO9515131

(56) Entgegenhaltungen:
- EP-A- 0 146 491
- EP-A- 0 255 797
- EP-A- 0 387 981
- EP-A- 0 451 875
- EP-A- 0 456 103
- EP-A- 0 551 543
- EP-A- 0 574 098
- DE-A- 3 320 395
- FR-A- 2 690 836
- GB-A- 2 210 707
- GB-A- 2 241 362
- US-A- 5 121 333

## Beschreibung

Die Erfindung betrifft ein Verfahren zur Herstellung von Endoprothesen, insbesondere von individuell konstruierten Implantaten und Augmentaten für die rekonstruktive Kopfchirurgie, bei welchem mittels Computertomographie der Datensatz eines dreidimensionalen Istmodells der vorhandenen Knochenstruktur eines Patienten akquiriert wird, das derart ermittelte Istmodell vom Datensatz eines vorliegenden oder mittels Computertomographie akquirierten dreidimensionalen Sollmodells in einem Rechner subtrahiert wird und aus der Differenz der Datensätze eine rechnerinterne Vorlage für die Endoprothese gebildet wird, die am Bildschirm des Rechners durch eine interaktive Manipulation der Daten an die anatomischen Besonderheiten des Patienten angepaßt wird und deren Datensatz abschließend zur rechnergesteuerten Fertigung der Endoprothese verwendet wird.

Ähnliche Verfahren, allerdings ohne korrigierende Anpassung an die anatomischen Besonderheiten des Patienten durch eine interaktive Manipulation des Datensatzes, sind beispielsweise aus der EP 0 255 797 B1, der EP 0 093 869 A1, der EP 0 146 491 A2 oder der EP 0 097 001 A1 bekannt. Mit den nach diesem Stand der Technik bekannten Verfahren ist es bei Verwendung entsprechend hochauflösender bildgebender Verfahren möglich, solche Endoprothesen verhältnismäßig genau an die vorhandene und zu ergänzende Knochenstruktur des Patienten anzupassen.

Bei der Herstellung von Endoprothesen zur Wiederherstellung oder zum Auffüllen von knöchernen Defekten im Bereich des Gesichts- oder Gehirnschädels, insbesondere bei der Herstellung von Augmentaten zur Ergänzung von atrophierten Alveolarfortsätzen für die Bereitstellung einer geeigneten Zahnprothesenbasis, reicht eine genaue Anpassung der Anlagerungsfläche der Endoprothese an die Oberfläche der noch vorhandenen und zu ergänzenden Knochenstruktur indessen nicht aus. Selbst bei einer sehr genauen Anpassung der Endoprothese an die vorhandene Oberfläche treten z.B. im Bereich der Austrittsstellen der die Nerven aufnehmenden Knochenkanäle oder im Falle fortgeschrittener Atrophie im Bereich der freigelegten Knochenkanäle schmerzhafte Druckerscheinungen auf.

Aus diesem Grunde muß die Oberfläche der Endoprothese im Kontaktbereich zu nervalen Strukturen abweichend von der direkt abgeleiteten geometrischen Form gestaltet werden, indem sie in diesem Oberflächenbereich zurückgenommen wird. Dabei müssen die zurückgenommenen Flächenbereiche mit sanft gerundeten Übergängen in die benachbarten, nicht zurückgenommenen Flächenbereiche übergehen, in denen die Endoprothese an der vorhandenen Knochenstruktur abgestützt ist.

Eine ähnliche korrigierende Anpassung der Endoprothese an die anatomischen Besonderheiten der vorhandenen Knochenstruktur ist notwendig, wenn die miteinander in Kontakt kommenden Oberflächen der vorhandenen Knochenstruktur und der Endoprothese Hinterschneidungen und/oder Vorsprünge aufweisen, die beim Einsetzen der Endoprothese im Wege sind. Auch solche den operativen Eingriff störenden Hinterschneidungen und/oder Vorsprünge müssen vor der Operation durch eine Anpassung der Endoprothese beseitigt werden.

Aussparungen in den herzustellenden Endoprothesen sind schließlich auch im Bereich von anderen, nicht knöchernen anatomischen Strukturen des Kopfes erforderlich, z.B. im Bereich der Nasen- oder Augenhöhlen, der Nasen-Nebenhöhlen, des Gehörganges, des Mittel- oder Innenohres sowie der Schädelbasis und insbesondere des Endokraniums.

Die oben erläuterten korrigierenden Anpassungen und Aussparungen an Endoprothesen sind nach dem Stande der Technik nur durch eine sehr arbeitsaufwendige manuelle Nachbearbeitung möglich, beispielsweise durch Schnitzen oder Fräsen. Diese Nachbearbeitung von Hand erfordert viel Erfahrung und Fingerspitzengefühl und führt bei Fehlanpassungen zur Unbrauchbarkeit der Endoprothese.

Aus der EP 0 093 869 A1 ist ein Verfahren für die Herstellung einer in den Oberschenkelknochen einsetzbaren Hüftgelenkprothese bekannt, bei welchem die Anpassung der Konturen der Endoprothese an die anatomischen Besonderheiten des Patienten am Bildschirm des Rechners mittels eines Deckstiftes oder eines elektronischen Schreibstiftes vorgenommen wird. Mit diesen Hilfsmitteln werden die die einzelnen Schichtbilder der Endoprothese repräsentierenden Daten entsprechend verändert (manipuliert) und gespeichert. Aus den gespeicherten Daten der auf diese Art und Weise manipulierten Schichtbilder kann der Rechner dann den Datensatz für ein dreidimensionales Modell errechnen. Anhand dieses Datensatzes wird dann die Prothesen-Fertigungsmaschine bei der Fertigung der Endoprothese gesteuert.

Die nach diesem Stand der Technik bekannte, rechnergestützte Konstruktion und Anpassung von Endoprothesen ist sehr arbeitsaufwendig und umständlich und ermöglicht allenfalls eine grobe Anpassung der Endoprothese an die patientenseitig vorhandenen Knochenstrukturen. Eine solche Grobanpassung ist z.B. für orthopädische Hüftgelenkprothesen völlig ausreichend. Dort ist nämlich die sich an der Innenwand des durch Ausschaben von Spongiosa künstlich geschaffenen Knochenhohlraumes anlegende Außenfläche der Prothese mit Stufen versehen, die eine verbesserte Einleitung der statischen und dynamischen Kräfte in den Knochen bewirken sollen. Aus diesem Grunde brauchen dort die Konturen der Stufen in den aufeinander folgenden horizontalen Ebenen nur etwa dem Verlauf der Grenzfläche zwischen Spongiosa und Kompakta angepaßt zu werden.

Im Gegensatz zu orthopädischen Prothesen kommt es bei Endoprothesen für die Kopfchirugie weniger auf die Einleitung von statischen und dynamischen Kräften und stattdessen mehr auf eine korrigierende Anpassung der Oberflächenverläufe unter Freilassung von empfindlichen nervalen Strukturen der Knochenoberfläche an. Bei dieser korrigierenden Anpassung kommt man mit einer einfachen Schichtdarstellung und einer von Hand vorgenommenen Veränderung der Konturen der einzelnen Schichtbilder nicht mehr aus. Insbesondere wäre es bei einer schichtweise vorgenommen Korrektur der Konturen der einzelnen Schnittlinien außerordentlich schwierig, die zurückgenommenen Flächenbereiche mit sanft gerundeten Übergängen in die benachbarten, nicht zurückgenommenen Flächenbereiche übergehen zu lassen, in denen die Endoprothese an der vorhandenen Knochenstruktur abgestützt ist.

Es ist deshalb Aufgabe der Erfindung, das Verfahren der eingangs genannten Art dahingehend weiterzubilden, daß der für die korrigierende Anpassung der Oberflächen der Endoprothese erforderliche Arbeitsaufwand verringert wird und automatisch für sanft gerundete Übergänge zwischen den zurückgenommenen Flächenbereichen und den benachbarten, nicht zurückgenommenen Flächenbereichen gesorgt wird.

Zur Lösung dieser Aufgabe schlägt die Erfindung ausgehend von dem Verfahren der eingangs genannten Art vor, daß die Datensätze des Istmodells und des Sollmodells in die Daten einer die Begrenzungsflächen der Modelle durch an Stützpunkten orientierte Spline- und Bézierfunktionen beschreibenden CAD-Freiformflächengeometrie umgesetzt und auf dem Bildschirm des Rechners übereinanderliegend abgebildet werden und daß anhand dieser Abbildung Teilbereiche der Grenzfläche des Istmodells durch Stützpunktverschiebung in Richtung auf das Volumen des Sollmodels zurückgenommen werden.

Die durch das Verschieben von Teilbereichen der Grenzfläche des Istmodells rechentechnisch gebildeten Hohlräume werden bei der mechanischen Herstellung des Knochenimplantats aus dem Volumen des Sollmodells abgetragen. Somit befinden sich bei der späteren Implantation des Augmentates diese Hohlräume an genau den Stellen, wo die Anpassung vorgenommen worden ist.

Das Verfahren gemäß der Erfindung hat den Vorteil, daß der Arbeitsaufwand für die korrigierende Anpassung auch bei sehr komplizierten Strukturen außerordentlich gering ist und daß gewissermaßen automatisch bei jedem Anpassungsvorgang die Übergangsbereiche zwischen den zurückgenommenen Flächenbereichen und den nicht zurückgenommenen Flächenbereichen sanft gerundet werden.

Das ist darauf zurückzuführen, daß beim Verfahren gemäß der Erfindung die Begrenzungsflächen der Modelle in einer Freiformflächengeometrie dargestellt werden, bei der die Flächen durch an Stützpunkten orientierte Spline- und Bézierfunktionen beschrieben werden. Dadurch werden nämlich die Begrenzungsflächen gewissermaßen zu kohärenten, räumlichen Gebilden, deren örtlich an Stützpunkten vorgenommene Manipulationen Auswirkungen auf alle an diesem Stützpunkt angrenzenden Flächen haben, wobei automatisch dafür Sorge getragen ist, daß die durch die Stützpunktverschiebung geänderten Flächenbereiche kontinuierlich und sanft gerundet in die benachbarten, nicht zurückgenommenen Flächenbereiche übergehen.

Gerade die Schaffung dieser sanften Übergänge ist bei Endoprothesen für die Kopfchirurgie außerordentlich wichtig und mit den nach dem Stande der Technik für diesen Zweck bekannten Maßnahmen nur schlecht und mühsam zu bewerkstelligen.

Die endgültige Gestaltung und Feinanpassung des durch die Zurücknahme entstandenen neuen Grenzflächenverlaufes wird zweckmäßig durch Spiegelung, Dehnung, Drehung oder Glättung unterstützt. Diese Manipulationsfunktionen machen es möglich, auch die abschließende Feinanpassung der Prothese bereits im Rechner an dem rechnerinternen Modell vorzunehmen, so daß bei der Fertigung der Prothese auch die Feinanpassungen schon berücksichtigt sind.

Das Verfahren gemäß der Erfindung eignet sich besonders für die Herstellung von Implantaten oder Augmentaten in der Kieferchirurgie. Es läßt sich aber auch für andere sensible Bereiche des Knochenbaus anwenden, beispielsweise zur Konstruktion individueller Implantate von Schädelteilen zur Behebung knöcherner Kontinuitätsdefekte nach temporoparietaler Trepanation.

Das Verfahren gemäß der Erfindung weist insbesondere den Vorteil auf, daß die Endoprothese unmittelbar mit allen zu berücksichtigenden Besonderheiten gefertigt werden kann. Eine Nacharbeit oder manuelle Feinanpassung ist nicht mehr erforderlich. Ein weiterer Vorteil besteht darin, daß die Behandlungszeit beim operativen Eingriff deutlich verkürzt werden kann.

Weitere Vorteile ergeben sich, wenn für die Datenakquisition ein hochauflösender Computertomograph mit helikaler Datenerfassung verwendet wird. Bei diesem bildgebenden Verfahren wird der datenmäßig zu erfassende Körperteil des Patienten durch eine schraubenförmige Wendel erfaßt, deren Steilheit durch den Tischvorschub je Umdrehung der Röntgenröhre vorgegeben ist.

Das gesamte von der Wendel erfaßte Patientenvolumen kann nachfolgend entsprechend herkömmlicher computertomographischer Darstellung erneut schichtweise rekonstruiert werden, wobei der Abstand dieser rekonstruierten Schichten nicht durch die Wendelsteigung vorgegeben ist, sondern beispielsweise auch kleiner gewählt werden kann.

Im Hinblick auf das Verfahren der eingangs genannten Art, bei welchem die Exaktheit der geometrischen Anpassung der Endoprothese an die knöcherne Struktur des Patienten lediglich durch die Validität der aufgearbeiteten computertomographischen Daten limitiert wird, ergeben sich durch die Verwendung eines solchen Computertomographen mit helikaler Datenakquisition zwei wesentliche unverzichtbare Vorteile gegenüber anderen computertomographischen Akquisitionsverfahren:
1. Die Schnelligkeit der Datenaufnahme von ca. 20 sek., quasi während eines Atemzuges (single breathhold), gewährleitet die Minimierung von patientenseitigen Bewegungsartefakten, die im nachfolgenden Rekonstruktionsprozeß nur schwerlich erkennbar und korrigierbar wären.
2. Die Erfassung eines Volumens anstelle von einzelnen Flächen (Schichten) erlaubt die rechnerinterne Rekonstruktion eines Geometriemodells bei bestmöglicher Erfassung von Zwischenschichtbereichen; es wird im Gegensatz zu konventionellen computertomographischen Techniken ein vollständiger oder - im Falle angrenzender oder überlappender Schichtführungen bei einem geometrisch gleichsam bikonkaven Röntgenstrahlbündel - ein nach zentral zunehmender Informationsverlust vermieden.

Ein Ausführungsbeispiel des Verfahrens gemäß der Erfindung wird im folgenden anhand der Zeichnungen näher erläutert. Es zeigen:
- Fig. 1: schematisch die Verfahrensschritte des Verfahrens gemäß der Erfindung in einem Blockdiagramm;
- Fig. 2: eine Gerätekonfiguration für die Durchführung des Verfahrens gemäß der Erfindung;
- Fig. 3: die idealisierte Freiformflächengeometrie eines atrophierten Unterkiefers als Istmodell in Perspektive;
- Fig. 4: die idealisierte Freiformflächengeometrie eines Vergleichsmodells als Sollmodell in der Perspektive;
- Fig. 5: die Darstellung der Überlagerung der Figuren 3 und 4 auf dem Bildschirm;
- Fig. 6: die Darstellung eines Vertikalschnittes entlang der Linie A - A in Fig. 5.

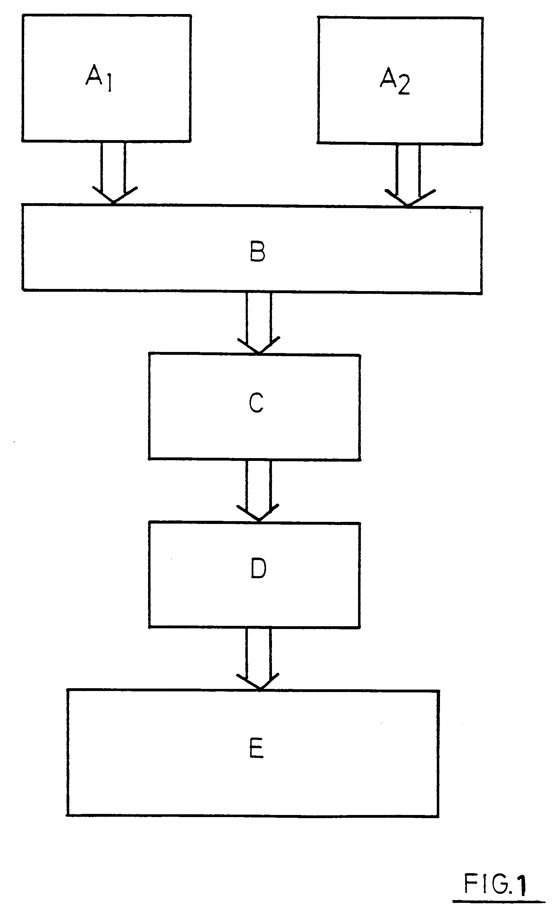

In Fig. 1 ist schematisch der Ablauf des Verfahrens zur Herstellung einer Endoprothese gemäß der Erfindung dargestellt. Im Verfahrensschritt A1 wird zunächst der Datensatz des Istmodells am Unterkiefer des zu behandelnden Patienten computertomographisch akquiriert. Zugleich wird an der Position A2 der Datensatz des Sollmodells zur Verfügung gestellt. Dieser Datensatz befindet sich entweder auf einem geeigneten Speichermedium oder wird ebenfalls computertomographisch von einem körperlich vorhandenen Sollmodell akquiriert.

Im Verfahrensschritt B werden die Datensätze des Istmodells und des Sollmodells rechentechnisch in die Dateneinheit CAD-Freiformflächengeometrie umgesetzt. Dabei handelt es sich um eine an Stützpunkten orientierte, dreidimensionale Darstellung der Begrenzungsflächen der Modelle, die durch numerische Spline- und Bézierfunktionen beschrieben werden. Die Flächengebilde in dieser Darstellung zeichnen sich dadurch aus, daß sie durch interaktive CAD-Modellier- und Manipulationsverfahren leicht handhabbar sind.

Im sich anschließenden Verfahrensschritt C werden die umgesetzten Datensätze des Istmodells und des Sollmodells übereinanderliegend auf dem Bildschirm abgebildet. Anhand dieser Abbildung werden Teilbereiche der Grenzfläche des Istmodells durch ein interaktives CAD-Modellier- und Manipulationsverfahren in Richtung auf das Volumen des Sollmodells verschoben. Dadurch werden in den Bereichen der Endoprothese, in denen eine Anlage an der vorhandenen Knochenstruktur unerwünscht ist, Ausnehmungen und Hohlräume erzeugt. Ebenso können Vorsprünge und/oder Hinterschneidungen der herzustellenden Endoprothese weggenommen werden, die im voraus erkennbar beim Einsetzen der Endoprothese Schwierigkeiten machen würden.

Im Verfahrensschritt D wird schließlich die Differenz der Daten von Ist- und Sollmodell gebildet und auf diese Weise ein Datensatz erzeugt, der als Vorlage für die rechnergestützte Fertigung der Endoprothese dienen kann.

Anhand dieses Datensatzes wird schließlich im Verfahrensschritt E mit Hilfe einer rechnergesteuerten Fertigungseinheit die fertige Endoprothese hergestellt.

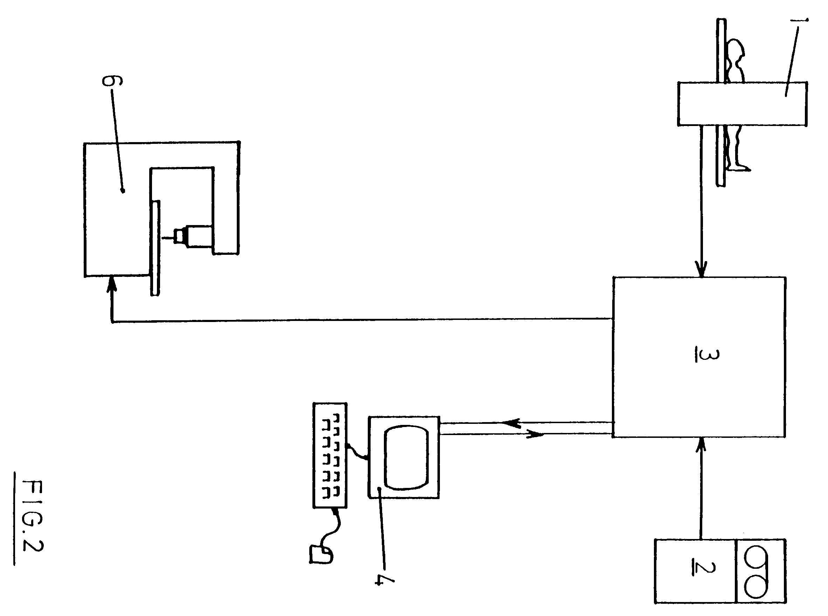

Die in Fig. 2 dargestellte Gerätekonfiguration weist einen Computertomographen 1 mit helikaler Datenakquisition auf. Dieser Computertomograph dient für die Akquirierung der Daten des Istmodells am Patienten. Die Daten des Sollmodells werden entweder ebenfalls in dem Computertomographen 1 anhand eines körperlich vorhandenen Sollmodells akquiriert oder stehen in einem geeigneten Daten-Massenspeicher 2 zur Verfügung. Zur Umsetzung der akquirierten oder zur Verfügung gestellten Datensätze von Istmodell und Sollmodell in die Daten einer CAD-Freiformflächengeometrie und zur anschließenden Manipulation dieser Daten dient ein entsprechend leistungsfähiger Rechner 3, der mit einem Bildschirm 4 und der üblichen digitalen und/oder graphischen Eingabeeinheit versehen ist.

Auch die abschließende Differenzbildung für die Erzeugung der rechnerinternen Vorlage für die herzustellende Endoprothese erfolgt in dem Rechner 3.

Abschließend wird die Endoprothese in einer rechnergesteuerten Fertigungseinheit 6 hergestellt.

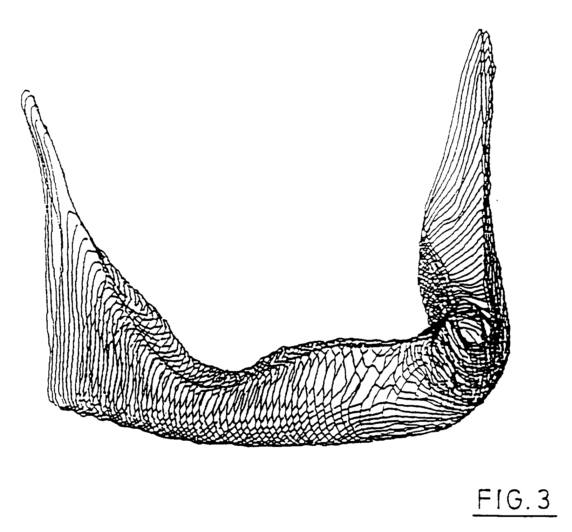

Fig. 3 zeigt einen Ausdruck einer Darstellung des Istmodells, wie es beim Verfahrensschritt C auf dem Bildschirm 4 dargestellt wird.

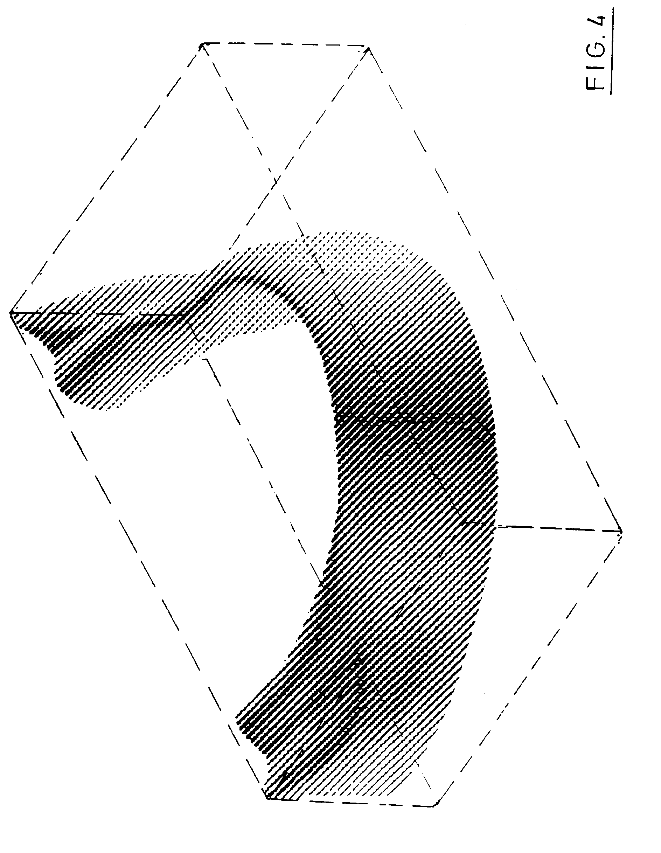

Fig. 4 zeigt in der gleichen Darstellung das Sollmodell.

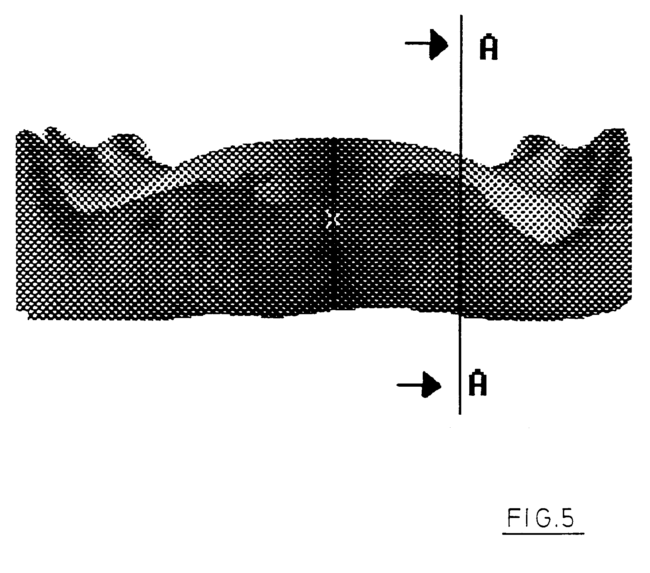

In Fig. 5 sind das Istmodell gemäß Fig. 3 und das Sollmodell gemäß Fig. 4 übereinanderliegend dargestellt. Bei dieser Darstellung auf dem Bildschirm 4 werden zweckmäßig beide Modelle in unterschiedlichen Farben dargestellt, um sie besser unterscheiden zu können. Durch die beiden übereinander dargestellten Modelle können beliebige Schnitte gelegt werden, anhand derer an den kritischen Stellen die Anpassung und anschließende Feinanpassung vorgenommen werden. Die Anpassung erfolgt im wesentlichen durch Stützpunktverschiebung. Die Feinanpassung erfolgt durch geometrische Manipulationsfunktionen (Spiegelung, Dehnung, Drehung, Rundung, Glättung usw.). Die Anpassung erfolgt derart, daß Höhe und Lage in Richtung der sagittalen und transversalen Ebenen sowie die Aussparungen von Austrittsbereichen der sensiblen Unterkiefernerven der Gestalt der zu erzeugenden Endoprothese angepaßt sind. Die wohlgerundete Form und der geschwungene Verlauf der sagittalen Ebenenorientierung (Kompensationskurve) der zu erzeugenden Endoprothese ergeben sich bereits vorab durch das in Fig. 4 abgebildete Sollmodell.

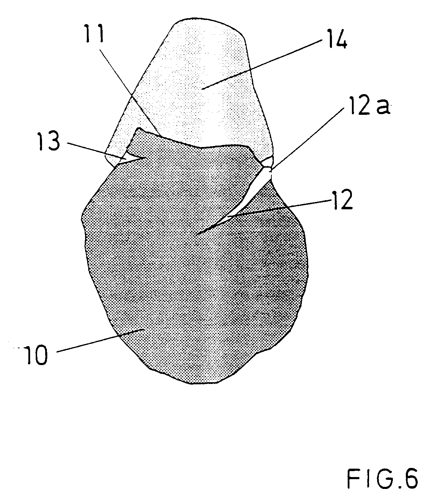

Ein Schnitt entlang der Linie A-A in Fig. 5 ist in Fig. 6 dargestellt. Dort ist zur besseren Erkennbarkeit der Querschnitt des Istmodells 10 mit seiner Grenzfläche 11, dem Knochenkanal 12 und einer Hinterschneidung 13 dunkel schraffiert dargestellt. Der Querschnitt des Sollmodells 14 ist demgegenüber hell schraffiert dargestellt. Wie aus Fig. 6 erkennbar, ist die Grenzfläche 11 des Istmodells 10 im Bereich des Austrittes 12a des Knochenkanals 12 und der Hinterschneidung 13 in das Volumen des Sollmodells 14 verschoben worden. Die durch die Verschiebung freigewordenen Querschnittsflächen des Sollmodells sind nicht schraffiert. Nach der Differenzbildung von Istmodell 10 und Sollmodell 14 entstehen im Bereich dieser nichtschraffierten Flächen Ausnehmungen oder Höhlungen, in deren Bereich die hergestellte Endoprothese nicht an der Oberfläche der Knochenstruktur des Patienten (= Istmodell) anliegt.

## Patentansprüche

1. Verfahren zur Herstellung von Endoprothesen, insbesondere von individuell konstruierten Implantaten und Augmentaten für die rekonstruktive Kopfchirurgie, bei welchem mittels Computertomographie der Datensatz eines dreidimensionalen Istmodells (10) der vorhandenen Knochenstruktur eines Patienten akquiriert wird, das derart ermittelte Istmodell vom Datensatz eines vorliegenden oder mittels Computertomographie akquirierten dreidimensionalen Sollmodells (14) in einem Rechner subtrahiert wird und aus der Differenz der Datensätze eine rechnerinterne Vorlage für die Endoprothese gebildet wird, die am Bildschirm des Rechners durch eine interaktive Manipulation der Daten an die anatomischen Besonderheiten des Patienten angepaßt wird und deren Datensatz abschließend zur rechnergesteuerten Fertigung der Endoprothese verwendet wird, **dadurch gekennzeichnet,** daß die Datensätze des Istmodells (10) und des Sollmodells (14) in die Daten einer die Begrenzungsflächen der Modelle durch an Stützpunkten orientierte Spline- und Bézierfunktionen beschreibenden CAD-Freiformflächengeometrie umgesetzt und auf dem Bildschirm des Rechners übereinanderliegend abgebildet werden und daß anhand dieser Abbildung Teilbereiche der Grenzfläche (11) des Istmodells (10) durch Stützpunktverschiebung in Richtung auf das Volumen des Sollmodells (14) zurückgenommen werden.

2. Verfahren nach Anspruch 1, dadurch gekennzeichnet, daß die endgültige Gestaltung und Feinanpassung des durch die Zurücknahme entstandenen neuen Grenzflächenverlaufs durch Spiegelung, Dehnung, Rundung oder Glättung unterstützt wird.

3. Verfahren nach einem der Ansprüche 1 oder 2, dadurch gekennzeichnet, daß für die Datenakquisition ein hochauflösender Computertomograph (1) mit helikaler Datenerfassung verwendet wird.

## Claims

1. Process for producing endoprostheses, in particular individually constructed implants and augments for reconstructive head surgery, in which the data set of a three-dimensional actual model (10) of the existing bone structure of a patient is acquired by means of computer tomography, the actual model ascertained in that way is subtracted in a computer from the data set of an existing or computer-tomographically acquired, three-dimensional reference model (14), and a computer-internal model for the endoprosthesis is formed based on the difference of the data sets, said computer-internal model being adapted on the video screen of the computer by interactive manipulation of the data to the particular anatomical features of the patient and the data set thereof being finally used for the computer-controlled manufacture of the endoprosthesis, characterised in that the data sets of the actual model (10) and of the reference model (14) are converted into the data of a CAD free-form surface geometry describing the limiting surfaces of the models by spline and Bezier functions oriented on points of support, and shown in superimposed relationship on the video screen of the computer, and that based on said imaging partial regions of the interface (11) of the actual model (10) are recessed by support-point displacement in the direction towards the volume of the reference model (14).

2. Process according to claim 1 characterised in that final shaping and fine adaptation of the new configuration of the interface created by recession is supported by reflecting, expanding, rounding or smoothing.

3. Process according to one of claims 1 and 2 characterised in that a high-resolution computer tomograph (1) with helical data collection is used for data acquisition.

## Revendications

1. Procédé pour la fabrication d'endoprothèses, en particulier d'implants et augments individuellement construits pour la chirurgie reconstructive de la tête où, au moyen d'une tomographie par ordinateur du groupe de données d'un modèle tridimensionnel réel (10), la structure osseuse présente d'un patient est acquise, le modèle réel ainsi obtenu est soustrait, dans un calculateur, du groupe de données d'un modèle de consigne tridimensionnel (14) présent ou acquis au moyen d'une tomographie par ordinateur et, de la différence des groupes de données est formé un dessin interne au calculateur pour l'endoprothèse, qui est adapté, sur l'écran du calculateur, par une manipulation intéractive des données, aux particularités anatomiques du patient et dont le groupe de données est utilisé finalement pour la fabrication commandée par ordinateur de l'endoprothèse, caractérisé en ce que les groupes de données du modèle réel (10) et du modèle de consigne (14) sont transformés en données d'une géométrie de surface en formage libre - CAD orienté vers les points d'appui et décrivant les fonctions de Spline et Bézier et sont reproduits sur l'écran de l'ordinateur et, en fonction de cette reproduction, des zones partielles de la surface limite (11) du modèle réel (10) sont retirées par déplacement des points d'appui dans la direction du volume du modèle de consigne (14).

2. Procédé selon la revendication 1, caractérisé en ce que la configuration finale et l'adaptation précise du nouveau tracé de la surface limite, résultant du retrait, est aidé par réflexion, extension, arrondissement ou lissage.

3. Procédé selon la revendication 1 ou 2, caractérisé ce que, pour l'acquisition de données, on utilise une tomographie par ordinateur (1) à haute résolution avec saisie de données en hélice.
